# EUROPEAN PATENT APPLICATION

(11) **EP 1 703 282 A1**
(43) Date of publication of application: **20.09.2006**
(21) Application number: 05090066.1
(22) Date of filing: 15.03.2005
(51) Int. Cl.: G01N 33/68

(54) **Method for ante mortem diagnosis of a TSE disease**

(71) Applicant: BRD, vertreten durch das BM für Gesundheit und Soziale Sicherung, Letztvertreten durch den Präsidenten des Robert-Koch-Inst., 13353 Berlin (DE)
(72) Inventor: Baier, Michael,Dr., 10435 Berlin (DE); Bamme, Theresa, 10783 Berlin (DE); Riemer, Constanze, Dr., 13467 Berlin (DE)
(74) Representative: Ziebig, Marlene

(57) **Abstract**

The invention relates to a method for ante mortem diagnosis of a transmissible spongiform encephalopathy (TSE) disease in a mammal comprising the incubation of a sample of a body fluid taken from the mammal with substances being specific to a surrogate marker of TSE, the detection of the specific incubation products, the determination of the surrogate marker concentration and based on it the diagnosis of TSE, wherein the diagnosis of TSE is performed on a mammal showing no symptoms of TSE, wherein the body fluid which is taken from the mammal is selected from the group consisting of blood, serum, plasma, saliva, and urine, and wherein substances being specific to CXCL13 are used for diagnosis of TSE. Object of the invention is also a method for ante mortem differential diagnosis of a TSE disease, and the use of substances being specific to CXCL13 in these methods.

## Description

The present invention relates to a method for ante mortem diagnosis of a transmissible spongiform encephalopathy (TSE) disease in a mammal, a method for ante mortem differential diagnosis of a TSE disease in a mammal, and the use of substances being specific to CXCL13 in the aforementioned methods.

Transmissible spongiform encephalopathies (TSEs), such as scrapie in sheep, bovine spongiform encephalopathy (BSE) in cattle, and Creutzfeldt-Jakob disease (CJD) in humans, are infectious and invariably fatal neurodegenerative disorders of the central nervous system. The neuropathology of these TSE diseases is usually linked to the appearance of an abnormal insoluble and protease-resistant form of a normal host-encoded protein, the prion protein (PrP). TSEs in general are characterized by a reactive gliosis and the subsequent degeneration of neuronal tissue. The activation of glial cells which precedes neuronal death is likely to be caused by the deposition of large amounts of PrP^{Sc} in the brain. PrP^{Sc} (herein, sc denote scrape) or PrP^{res} (herein, res denotes resistant) refer to the disease-associated abnormal form of PrP of any TSE disease, being characterized by a high beta-sheet content in contrast to the predominantly alpha helical fold of normal PrP. Following the BSE epidemic in the United Kingdom and the emergence of new variant CJD (vCJD), the development of diagnostic methods that permit the detection of TSEs in vivo has become a matter of great scientific and public importance.

Prior art methods are focused on the post mortem diagnosis of TSE diseases. Except for histopathology, prior art methods are based upon the detection of accumulated, protease-resistant PrP^{Sc} in specific regions of the central nervous system (CNS) by immuno-histochemistry, western blotting and ELISA.

A test is known which PrP^{Sc} detection system is a two site non-competitive immunometric procedure using two different monoclonal antibodies (E.G. & G. Wallace Ltd.). The reading signal is generated by DELFIA technology. Unfortunately, the test takes nearly 24 hours to be carried out, and exhibits a low sensitivity and specificity.

Further immunotests are known, e.g. based on a western blotting procedure (Prionics Check), high throughput chemiluminescent ELISA (Enfer Ltd.), and sandwich immunoassays (CEA) using one or more monoclonal or polyclonal antibodies for PrP^{Sc}. Even though the diagnosis rate, sensitivity and specificity are increased, all prior art procedures require the withdrawal of samples from brain for the detection of PrP^{Sc}. Therefore, these methods are particularly disadvantageously because of their exclusive application on the dead mammal.

Several studies have been carried out to investigate disease-associated alterations of gene expression in scrapie-infected brain tissue to provide a framework for the development of interventional and diagnostic strategies as well as to define therapeutic targets. For example, among 1.200 clones generated by suppression subtractive hybridization technique, 19 clones displayed upregulated signal intensities in brain, such as increased mRNA levels of CXCL13 (Riemer et al., 2000, J. Virology 74, 10245-10248). Another large-scale array for scrapie-related gene expression profile alterations in the cortex, medulla, and pons covering more than 11.000 functionally characterized sequences and sequence tags identified in total 114 genes with altered mRNA levels (Riemer et al., 2004, BBRC 323, 556-564). 86 of 114 genes are upregulated in the brain of infected mammals.

However, it is not predictable so far whether the gene overexpression in brain can also be exhibited in other body fluids excepting those of CNS, and vice versa. In both cases, the blood-brain barrier has to be overcome, for instance in consequence of a damage caused by a progressing disease.

A few markers have been considered in the prior art for ante mortem diagnosis of a TSE disease. It was recently reported that significantly increased levels of serum S100 β protein can be observed in patients with genetic and sporadic CJD, and in an animal model for TSEs, maybe resulting form astroglial activation, astroglial destruction, or a breakdown of the blood-brain barrier. Actually, the elevated serum S100 β levels were only demonstrated in terminally ill animals which also show unambiguous clinical symptoms (Beekes et al, 1999, J. Infectious Diseases 180, 518-520).

A similar late increase of protein level was found for the serum amyloid P.

Additionally, a spectroscopic method has been described for the detection of complex changes in serums of PrP^{Sc}-infected mammals (Lasch et al., 2003, Anal. Chem. 75, 6673-6678). Once more, the late diagnosis of TSE, i.e. in terminal stages of the disease, is of disadvantage.

Therefore, the technical problem forming the basis of the present invention is to provide a method for the diagnosis of a TSE disease on a mammal which can be performed ante mortem and at a stage of the TSE disease in which the mammal is free of symptoms.

Surprisingly, it has been found that the chemokine CXCL13 is able to overcome the blood-brain barrier in an infected mammal and at a stage of a TSE disease which is not correlated with apparent TSE symptoms. Therefore, the present invention provides a method for ante mortem diagnosis of a TSE disease in a mammal comprising the incubation of a sample of a body fluid taken from the mammal with substances being specific to a surrogate marker of TSE, the detection of the specific incubation products, the determination of the surrogate marker concentration and based on it the diagnosis of TSE, wherein the diagnosis of TSE is performed on a mammal showing no symptoms of TSE, wherein the body fluid which is taken from the mammal is selected from the group consisting of blood, serum, plasma, saliva, and urine, and wherein substances being specific to CXCL13 are used for diagnosis of TSE.

In the meaning of the invention, surrogate marker relates to any substance which is associated with a TSE disease without being the infectious agent itself. The method according to the present invention is based on the surrogate marker CXCL13. Advantageously, the occurrence of this surrogate marker is not narrowed to the brain and the cerebrospinal fluid. Whereas the exclusive location of the PrP^{Sc} marker in the CNS essentially brings about the killing of the subject, the possibility of a non-invasive procedure arises from detecting specific incubation products of the surrogate marker. Therefore, samples of body fluids, such as blood or serum, are easily taken from the mammal. The mammal is still alive, and it is not necessary that the mammal shows any symptoms related to TSE.

Among the symptoms of TSE are aggressiveness, anxiousness/jumpiness, ataxia, progressive dementia, and myoclonus. These symptoms are also referred to as clinical which are used for diagnosis of TSE at the clinical, i.e, terminal stage of disease prior to death. However, the infected mammal could not be identified during the stage of disease which is termed as pre-clinical or asymptomatic due to the lack of the aforementioned symptoms. Therefore, the pre-clinical diagnosis according to the present invention opens up new vistas concerning an early and purposive therapy currently to be developed.

The inventive method can be performed simply, fast and cost-efficiently. The provision of an diagnostic tool even reliable in absence of clinical symptoms is especially advantageous to prevent the slaughtering of entire populations, such as cattle herds, flocks of sheep, and wild game populations. In response to the appearance of single cases of clinical TSE, among the other mammals infected and uninfected ones may be differentiated by the present ante mortem diagnosis of TSE, thereby enabling culling.

Furthermore, the present invention is well-suited for screening of blood donors or patients prior to surgery invasion in order to prevent the transfer of infection via blood, blood products, or contaminated surgical instruments.

In detail, the present surrogate marker CXCL13, also known as B-lymphocyte chemoattractant (BLC) or B cell-attracting chemokine 1 (BCA-1), is a member of the CXC subtype of the chemokine superfamily. The gene encodes a putative protein of 109 amino acids including a 21 amino acid leader peptide plus an 88 amino acid mature segment. In general, CXCL13 is critical for secondary lymphoid tissue development and navigation of lymphocytes within the micro-compartments of these tissues. It mediates chemotactic response, preferably for lymphocyte homing of B and T cells, and CXCL13 is a chemoattractant for neutrophils. Therefore, it plays a potential role in the regulation of humoral immunity.

Surprisingly, the inventors have found that CXCL13 overexpression in CNS is reflected by noticeable enhanced concentration levels out of CNS which are already detectable at the asymptomatic stage of TSE disease. Secreted CXCL13 possesses a favorable stability and half-life in non-CNS body fluids. Advantageously, the increase in CXCL13 concentration is independent on both the mode of infection which comprises intraperitoneal, intracerebral, or oral uptake/ administration, and the type of infectious strain.

The sample of body fluid is withdrawn from a mammal to be examined following good medical practice. In the present invention, the sample of body fluid preferably consists of blood, serum, plasma, saliva, or urine, which are not contaminated with the infectious agent PrP^{Sc} causing TSE. The sample may be purified to remove disturbing substances, such as inhibitors for the formation of hydrogen bonds, or the surrogate marker CXCL13 can be concentrated in the sample. Downstream-processing and/or concentrating are preferably performed by the method of precipitation, dialysis, gel filtration, gel elution, or chromatography, such as HPLC or ion exchange chromatography. It is recommended to combine several methods for better yields.

The sample of the body fluid taken from the mammal is incubated with substances which are specific to CXCL13 or variants thereof. The homology between native CXCL13 and variants thereof amounts to at least 85 %, whereby the degree of variation is inevitably limited by the requirement of recognition by the specific substances. Possible alterations comprise deletion, insertion, substitution, modification and addition of at least one amino acid, or the fusion with another protein.

The term specific substances as used herein comprises molecules with high affinity to CXCL13 or variants thereof, preferably proteins or peptides, nucleic acids, or low molecular weight ligands. The proteins or peptides are preferably selected from the group consisting of antibodies, receptors, lectins, avidins, lipoproteins, glycoproteins, oligopeptides, peptide ligands, and peptide hormones. In an especially preferred embodiment of the invention, antibodies are used. The nucleic acids are preferably single or double stranded DNA or RNA, oligonucleotides, aptamers or Spiegelmers, and parts thereof. Preferred examples of low molecular weight ligands are biotin, biotin derivatives, and steroids. The specific substances according to the invention express a sufficient sensitivity and specificity to CXCL13 and variants thereof in order to ensure a reliable detection.

The specific substances can be labeled, in doing so the labeling depends on their inherent features and the detection method to be applied. Preferred specific incubation products of the inventive method are ligand-acceptor complexes consisting of specific substance and CXCL13, preferably antibody-CXCL13 complexes or aptamer-CXCL13 complexes.

Incubation denotes the contacting of specific substances with CXCL13 which can be realized without a chemical conversion, e.g. antibody-CXCL13 binding, or may involve a biochemical reaction, e.g. by an enzyme-CXCL13 complex. The accessibility of CXCL13 in the sample of body fluid may be improved by adding chemical solutions and/or applying physical procedures, e.g. impact of heat.

For the detection of the specific incubation products, the applied methods depend on the specific incubation products to be monitored and are well-known to the skilled artisan.

Along with the detection, the concentration of the specific incubation products and the concentration of the surrogate marker are determined. Therefor, the signal intensity of the incubation products is correlated with the signal intensity of a calibration curve enabling the meter-reading of a matching concentration of the incubation product. Preferably, the calibration curve is based on the Lambert-Beer equation if using UV/VIS coloring or luminescence. The concentration of the surrogate marker is subsequently calculated by considering the molar part of CXCL13 within the product complex. Preferably, the molar ratio of specific substance and CXCL13 is 1:1 which is present in antibody/CXCL13 complexes for instance, so that the molar concentration of the incubation products corresponds to the molar concentration of CXCL13.

TSE is diagnosed by comparing the concentration of CXCL13 in the mammal with known CXCL13 concentration levels of either an uninfected mammal or an infected mammal showing no symptoms of TSE. It shall be understood that the known concentrations are statistically proven, therefore representing a certain level or range, respectively. Any measured CXCL13 concentration which is higher than the CXCL13 concentration level of uninfected mammals indicates a TSE disease of the tested mammal, whereas that mammal is healthy at a CXCL13 concentration which is comparable to the concentration level of uninfected mammals. Contrary, a CXCL13 concentration being in the range of the CXCL13 concentration of infected, but asymptomatic mammals indicates a TSE disease of the tested mammal. Any concentration values exceeding the aforementioned range also confirm a TSE disease which should be actually accompanied by clinical symptoms. Lower CXCL13 concentrations than the aforementioned range confirm the absence of TSE in the mammal.

In an embodiment of the present invention, in the case of a TSE disease the CXCL13 concentration exceeds one and a half to four times, preferably twice to three times, the CXCL13 concentration in a sample of an uninfected mammal.

In another embodiment of the present invention, the TSE disease is scrapie, BSE, CJD, or vCJD.

Transmissible progressive neurodegenerative disorders belonging to TSE diseases are scrapie, a naturally occurring disease of sheep and goats, and the corresponding pathologies in humans (CJD, Gerstmann-Straeussler-Scheinker syndrome, fatal familial insomnia, and kuru), cattle (BSE), and other animals. In addition, disease pattern are in motion as represented by vCJD, maybe caused by altered infectious strains. Among the TSE diseases, scrapie, BSE, CJD, and vCJD are considered as particularly significant for health policy and nutrition. They are preferably diagnosed by the inventive method, although it can be used for diagnosis of any TSE disease.

In another embodiment of the present invention, the mammal being diagnosed is a human, cattle, sheep, goat, or deer being associated with the aforementioned TSE diseases of special relevance. In a preferred embodiment of the invention, bovine CXCL13 is used as surrogate marker.

In another embodiment of the present invention, the specific incubation products are detected by luminescence, VIS coloring, radioactive emission, electrochemical processes, or mass spectrometry.

Luminescence concerns the emission of light. Preferably, chemiluminescence, bioluminescence and photoluminescence are applied for the detection of specific incubation products in the inventive method. Chemiluminescence involves the emission of visible light as a result of a chemical reaction. In the present invention, either the specific substances to CXCL13 or the detecting agents of the incubation products are covalently labeled with a chemical compound. A preferred chemical label in the inventive method is luminol exhibiting an intensive blue chemiluminescence after oxidation, e.g. with peroxidase and H₂O₂. Photoluminescence, also known as fluorescence stimulation, is favorably caused by the absorption of photons, whereas bioluminescence requires the activity of luciferase. Both subtypes of luminescence are explained in detail in the advanced course of this specification.

VIS coloring denotes the visualization of any achromatic substance, such as the specific incubation products of the present invention, in order to be visible to the naked eye. Preferably, the intensity of coloring is measured by a photometer. In the present invention, the visible color is preferably produced by enzymatic catalysis of substrates, agglutination of silver particle, or colored latex particles. All labels underlying these reactions are associated in any manner to the incubation products of CXCL13.

Representing a favorite labeling in the inventive method, radioactive radiation of isotopes is measured by scintillation. The process of liquid scintillation involves the detection of beta decay within a sample via capture of beta emissions in a system of organic solvents and solutes referred to as the scintillation cocktail. The beta decay electron emitted by the radioactive isotope, such as ³H, ¹⁴C, ³²P, ³³P, and ³⁵S, in the sample excites the solvent molecule, which in turn transfers the energy to the solute. The energy emission of the solute (the light photon) is converted into an electrical signal by a photo-multiplier tube within a scintillation counter. The cocktail must also act as a solubilizing agent keeping a uniform suspension of the sample. Gamma ray photons often arise as a result of other decay processes (series decay) to rid the newly formed nucleus of excess energy. They have no mass and produce little if any direct ionization by collision along their path. Gamma photons are absorbed for detection and quantitation by one or more of three mechanisms: The Compton effect, the photoelectric effect, and pair production. A favorable gamma decay isotope of the present invention is ¹²⁵I.

Another favorite method for the detection of the specific incubation products of the present invention are enzyme electrodes. Therein, immobilized enzymes act as receptor and an electrochemical measuring system represents the inductor. The enzymes are immobilized either directly or indirectly to the specific substances for CXCL13. By the enzymatic attack of electrode-active substrates, a chemical signal is provided which is transformed into an electrical signal by means of a transducer. Either the substrates are located on an external solid phase in the opposite direction of the enzymes, or they are coupled to the specific substances. Preferred systems in the meaning of the invention are based on specific oxidases, such as glucose oxidase, additionally requiring co-enzymes to ensure the connection with electrochemical or optical sensor elements via redox mediators.

Furthermore, immunosensors are advantageously applied in the present invention. The energy of interaction between receptor (antibody) and immunogen (CXCL13 antigen) underlying immunosensors of the inventive method is transformed into an electrical signal by electrochemical, optical, calorimetric, or piezo-electric transducers, preferably by field effect transistors (FET). FET are electronic components controlling the current in the semiconductor material by the electrical field at the gate. The electrical field can be varied by the binding of molecules to the gate. Changes in dipole moments or a charge accumulation at immobilized biomolecules, such as antibodies, which are caused by ligands, such as antigens, are monitored by Bio-FET, preferably IMMUNFET. The antibodies may be of any type known in the art and are favorably immobilized.

The specific incubation products can also be detected by mass spectrometry, preferably MALDI-TOF. The incubation products are adsorbed in a suited matrix showing a high absorption at the excited laser wave length, thereby preventing the generation of fragment ions. The complex of specific incubation products remains stable by the gentle generation of ions enabling the separation from unbound specific substances, such as antibodies. The detection of time-of-flight mass spectrometers is based on a mass separation principle in high vacuum. To be considered are ionized species starting from the same position at the same time, being accelerated by means of a constant homogeneous electrostatic field. Their velocities are unambiguously related to their mass-to-charge ratio, and times of arrival at a detector directly indicate their masses.

In another embodiment of the present invention, antibodies are used as specific substances to CXCL13 and the incubation products are detected by the labeling of the antibodies, preferably by ELISA, RIA, FIA, SPIA, or western blotting.

Antibody denotes a polypeptide essentially encoded by an immunoglobulin gene or fragments thereof. According to the invention, antibodies are present as intact immunoglobulins or a number of well-characterized fragments. Fragments are preferably selected from the group consisting of F_{ab} fragments, F_{c} fragments, single chain antibodies (scFv), variable regions, constant regions, H chain (V_{H}), and L chain (V_{L}), more preferably F_{ab} fragments and scFv. Fragments, such as F_{ab} fragments and F_{c} fragments, can be produced by cleavage using various peptidases. Furthermore, fragments can be engineered and recombinantly expressed, preferably scFv.

There is a distinct number of specific antibodies against the antigen CXCL13 existing. In a preferred embodiment, the specific substance is represented by anti-CXCL13 antibodies which are described in the art. Antibodies are usually produced in mammal organisms when an immune response is caused by antigens being strange to the organism and having a molecular weight which exceeds 3.000 g/mol. In detail, both polyclonal and monoclonal antibodies are known which are directed to the murine or the human CXCL13 antigen. Favorable host species for antibody production comprise goat, rabbit, and mouse. Further polyclonal and monoclonal antibodies can be selected against CXCL13 originated form different species and fragments thereof. Popular techniques, such as the hybridoma technology, are well-known to the skilled artisan. The antibodies directed against CXCL13 are applied as specific substances in the inventive method.

The use of specific antibodies enables the diagnosis of a potential TSE disease on humans or animals, e.g. by an immunological test, preferably by means of solid phase immunoassays. Either the antigen CXCL13 or the antibody, preferably the antibody, is interlinked directly, i.e. covalently, or indirectly, i.e. non-covalently or via a spacer, with a label. Depending on the function of the label which should be either detected or recognized easily, the present invention differentiates between so-called detection labels and recognition labels. Immunological tests are ligand binding assays detecting an antigen by means of an antibody. Antibody and antigen, the latter being CXCL13 of 16 to 18 kDa in the present invention, form a complex called precipitate or agglutinate. Preponderantly, it is not directly detectable, therefore requiring an indicator reaction to become visible. Suited immunological detection procedures in the scope of the present invention may be divided by the type of indicator reaction.

In a preferred embodiment of the invention, enzyme-linked immunosorbent assays (ELISA) are used for the detection of the incubation products. Component of ELlSAs are enzymes which are bound to one partner of the immunological reaction. The tracer antigen (analyte derivative) of CXCL13 is preferably labeled in the competitive ELISA using a single capture antibody (herein after referred to as primary), whereas the antibody is preferably labeled in the non-competitive ELISA preferably comprising the precipitation of the antigen-antibody complex by a second antibody (herein after referred to as secondary) which is directed to another epitope of CXCL13 than the primary antibody. Complexes consisting of antigen and two antibodies are also called sandwich complexes. The detection comprises the subsequent enzymatic conversion of a substrate to a product, preferably a colored product, that is recognized by visual coloring, bioluminescence, fluorescence, or the measurement of electrical signals (enzyme electrode). Favorable enzymes for labeling in the present invention are known to the skilled artisan, such as peroxidase, G6PDH, CAT, GFP, GST, luciferase, β-galactosidase, and alkaline phosphatase.

Additionally preferred are radioactive immunoassays (RIA) utilizing radioactive isotopes which are either incorporated into an immune reagent during synthesis, preferably into tracer CXCL13, or subsequently coupled to an immune reagent of the assay, preferably to an antibody. Preferred radioactive isotopes in the inventive method are ³H, ¹⁴C, ³²P, ³³P, ³⁵S, and ¹²⁵I, and more preferred ¹⁴C, ³⁵S, and ¹²⁵I. A favorite method follows the competitive principle of binding. A constant amount of radioactive CXCL13 (tracer) and a variable amount of CXCL13 marker of the sample to be analyzed compete for a defined amount of antibody which is present in excess. The displacement of tracer is directly proportional to the marker concentration which can be evaluated by a calibration curve.

Antigens or antibodies, respectively, which are favorably labeled with fluorophores are used in fluoro immunoassays (FIA). Photoluminescence or fluorescence stimulation is caused by the absorption of energy, preferably provided by radiation, which is released again as photon with a shift in wave length of 30 to 50 nm, and within a period of approximately 10⁻⁸ seconds. Preferred fluorescence dyes in the method of the present invention are selected from the group of fluorescein, fluorescein isothiocyanat (FITC), ethidium bromide, acridine orange, and propidium iodide. Among these dyes, fluorescein and FITC are covalently bound to substances on protein basis.

The soluble particle immune assay (SPIA) utilizes the color change of silver particle as result of agglutination. Neither a secondary antibody nor an indicator reaction are required making it particularly useful in the scope of the present invention. Similarly favorably is the latex agglutination test using antibodies which are bound to colored latex particles. However, it requires a strong immobilization of CXCL13 to remove unbound and/or non-specifically bound antigens in previous washing steps.

In general, all methods for detection include intensive washing steps to separate unbound and/or non-specifically bound antigens from the CXCL 13/antibody complex. Furthermore, the experimental procedure of any detection method is well-known to those skilled in the art.

Another favorite detection method for specific incubation products of the invention is western blotting. Firstly, a gel is mixed and cast, samples previously prepared are loaded onto the gel and fractionated by electrophoresis. The proteins present in the polyacrylamide gel are blotted onto a nitrocellulose membrane to which antibodies may be applied to detect the specific protein of interest, CXCL13. Because the blotting process is not 100 % efficient, residual CXCL13 in the gel may be non-selectively stained using coomassie blue. Western blotting is simply performed and advantageously when an exact determination of the concentration is dispensable. Favorably, samples are prepared in such a way that the base level of CXCL13 present in an uninfected mammal is not detectable.

In a preferred embodiment of the invention, the antibodies for ELISA or western blotting are conjugated with reporter enzymes selected from the group consisting of peroxidase, CAT, GFP, GST, luciferase, β-galactosidase, and alkaline phosphatase.

The covalent linkage of an anti-CXCL13 antibody to an enzyme may be performed by different methods, such as the coupling with glutaraldehyde. Both, the enzyme and the antibody are interlinked with glutaraldehyde via free amino groups, and the by-products of networked enzymes and antibodies are removed. In another method, the enzyme is coupled to the antibody via sugar residues if it is a glycoprotein, such as the peroxidase. The enzyme is oxidized by sodium periodate and directly interlinked with amino groups of the antibody. Other enzyme containing carbohydrates can also be coupled to the antibody in this manner, however sometimes a loss in activity is observed due to the oxidation, e.g. a diminished activity of alkaline phosphatase. Enzyme coupling may also be performed by interlinking the amino groups of the antibody with free thiol groups of an enzyme, such as β-galactosidase, using a heterobifunctional linker, such as succinimidyl 6-(N-maleimido)hexanoate.

Peroxidase preferably originated from horseradish (herein after referred to as HRP) is capable of oxidizing several substrate, such as 2,2'-azino-di-3-ethylbenzthiazoline-6-sulfonic acid (ABTS) and tetramethylbenzidine (TMB). For example, the oxidation of ABTS requires the presence of H₂O₂. In a first step, a metastable radical cation is produced degrading in such a slow manner that its maximum of adsorption can be used for detection at 414 nm.

The inventive reporter enzyme in the detection system can be chloramphenicol acetyl transferase (CAT). In particular, CAT catalyzes the transfer of an acetyl residue from acetyl-CoA to chloramphenicol. Among other things, CAT has the advantage of being stable in body fluids with a half-life of about 50 hours. Advantageously, CAT has a good signal/background ratio.

Furthermore, the reporter enzyme is the green fluorescent protein (GFP), glutathione S-transferase (GST), and/or a fragment thereof. Advantageously, the absorption and emission maxima of the green fluorescent protein are similar to that of fluorescein, so that e.g. a GFP-positive sample can be detected using the same methods as with samples stained with fluorescein, i.e. under UV light or under a fluorescence microscope, for example.

Another advantageous reporter enzyme in the meaning of the invention is luciferase which catalysis of a redox reaction causes the emission of visible light. The energy gained during the luciferin/luciferase reaction is used for the electronic stimulation of an oxidation product of luciferin. Light is emitted by returning into the ground state. Luciferase is especially advantageous due to its high sensitivity.

By the activity of β-galactosidase, the achromatic substrate X-Gal is converted into a blue product which is visually recognized.

Alkaline phosphatase (AP) catalyzes the phosphate cleavage of bromo-4-chloro-3-indolyl phosphate (BCIP or X-phosphate). The resulting indoxyl residue tautomerizes into a ketone which subsequently dimerizes to blue indigo releasing hydrogen ions. Finally, the enhancer nitroblue tetrazolium (NBT) is reduced to a purple diformazan. This reporter enzyme is of particular benefit in western blotting since both dyes precipitate close to the alkaline phosphatase, thereby coloring the surrounding of bound CXCL13.

Furthermore, preferred reporter enzymes which can be used in the inventive method are GST as well as other fluorescent proteins, such as the red or the yellow protein, as well as the maltose-binding protein (MBP), G6PDH, and others.

In another preferred embodiment of the present invention, the antibodies for ELISA or western blotting are labeled with digoxigenin and non-covalently interlinked with anti-digoxigenin antibodies which are conjugated with reporter enzymes selected from the group consisting of peroxidase, CAT, GFP, GST, luciferase, β-galactosidase, and alkaline phosphatase.

In another preferred embodiment of the invention, the antibodies for ELISA or western blotting are biotinylated and non-covalently interlinked with streptavidin which is conjugated with reporter enzymes selected from the group consisting of peroxidase, CAT, GFP, GST, luciferase, β-galactosidase, and alkaline phosphatase.

Both preferred embodiments have in common that the detection label of the indicator reaction, i.e. a reporter enzyme, is not covalently bound to the anti-CXCL13 antibody, particularly the secondary antibody, therefore not being part of the immune precipitate or agglutinate, respectively. Here, the reporter enzyme is introduced into the indicator reaction in complex with a high affinity compound specifically targeting the recognition label of an antibody. Advantageous high affinity compounds to recognition labels can be any specific substance as previously defined in the course of the present specification, preferably antibodies herein after referred to as tertiary antibodies. It is to be understood that tertiary antibodies do not inevitably require a label for recognition, but may be directed against the secondary antibody itself, especially the F_{c} fragment. Advantageous labels in the inventive method are digoxigenin and biotin.

Digoxiginin (DIG) is a steroid hapten which does not occur in nature. Therefore, undesired side reactions are avoided by using the monoclonal anti-DIG antibody which is additionally characterized by a high sensitivity and specificity.

D-biotin is bound with a remarkably high affinity of 10⁻¹⁵ M by streptavidin from Streptomyces avidinii which is a homo-tetrameric protein containing a biotin binding site in each subunit of 159 amino acids.

Both, the monoclonal anti-DIG antibody and streptavidin can be conjugated with reporter enzymes, preferably aforementioned favorable enzymes, and by applying conjugation procedures as already described.

In a more preferred embodiment of the present invention, the antibodies are selected from the group consisting of goat polyclonal anti-human CXCL13 antibody AF801, biotinylated goat polyclonal anti-human CXCL13 antibody BAF801, mouse monoclonal anti-human CXCL13 antibody MAB801 clone 53610, mouse monoclonal anti-human CXCL13 antibody DCX130 part 892424, or mouse monoclonal anti-human CXCL13 antibody DCX130 part 892425.

These antibodies are commercially available at R&D Systems, Inc., Minneapolis, U.S.A. The codes stated above refer to catalog numbers. In detail, DCX130 refers to the Quantikine® kit which involves a human BLC/BCA-1/CXCL13 immunoassay. Part 892424 of this kit is a mouse monoclonal anti-human CXCL13 antibody which is coated on a 96 well polystyrene micro-plate, and part 892425 of this kit is a mouse monoclonal anti-human CXCL13 antibody conjugated to HRP in a preservative solution. It is to be understood that these antibodies can be used in the inventive method beyond their special embodiments. None of the antibodies shows a significant cross-reactivity or interference to other natural or recombinant human proteins, or to other natural or recombinant murine proteins including mouse CXCL13.

In addition, several polyclonal rabbit anti-human CXCL13 antibodies are known in the art. It shall be understood that this list is not exhaustive.

Furthermore, polyclonal and monoclonal antibodies against mouse CXCL13 can be used in the present invention. For example, the anti-murine CXCL13 antibodies are commercially distributed by R&D Systems, Inc., Minneapolis, U.S.A., preferably as component of the kit with the catalog number MCX130.

In another preferred embodiment of the present invention, a competitive ELISA is used comprising CXCL13 tracers which are conjugated with reporter enzymes selected from the group consisting of peroxidase, CAT, GFP, GST, luciferase, β-galactosidase, and alkaline phosphatase.

In another preferred embodiment of the invention, a competitive ELISA is used comprising CXCL13 tracers which are labeled with digoxigenin and non-covalently interlinked with anti-digoxigenin antibodies which are conjugated with reporter enzymes selected from the group consisting of peroxidase, CAT, GFP, GST, luciferase, β-galactosidase, and alkaline phosphatase.

In another preferred embodiment of the invention, a competitive ELISA is used comprising CXCL13 tracers which are biotinylated and non-covalently interlinked with streptavidin which is conjugated with reporter enzymes selected from the group consisting of peroxidase, CAT, GFP, GST, luciferase, β-galactosidase, and alkaline phosphatase.

The components of the competitive ELISA have been characterized in detail in the course of the present description. The prior teaching of the present invention is considered as valid and without restrictions for all embodiments of the competitive ELISA and its components.

Furthermore, the embodiments of prior teaching of the present invention are also applicable to the competitive ELISA and its components if expedient.

In another embodiment of the present invention, nucleic acids are used as specific substances to CXCL13 and the incubation products are detected by the labeling of the nucleic acids.

The term nucleic acid refers to a natural or synthetic polymer of single- or double-stranded DNA or RNA alternatively including synthetic, non-natural or modified nucleotides which can be incorporated in DNA or RNA polymers. Each nucleotide consists of a sugar moiety, a phosphate moiety, and either a purine or pyrimidine residue.

Preferred nucleic acids to be used in the inventive method are DNA aptamers and RNA aptamers which have been found to express a high affinity for a wide variety of molecules called targets. Target structures may comprise proteins, peptides, and small molecules, such as organic dyes, nucleotides, amino acids, vitamins, alkaloids etc. More preferred are RNA aptamers since the 2'-hydroxyl group available in RNA promotes a couple of intra- and intermolecular contacts, the latter being between molecules of the same sequence, different sequences, or between RNA and any other molecule which is not composed of RNA. These nucleic acid ligands can be identified by an efficient *in-vitro* selection procedure - the so-called SELEX process (systematic evolution of ligands by exponential enrichment). Since RNA is very susceptible to nucleolytic degradation in biological solutions, RNA aptamers should be chemically modified using phosphorothioates, locked nucleic acids, or Spiegelmers, for instance. L-RNA versions of aptamers called Spiegelmers are especially long-lived as they are essentially impervious to natural degradation processes. Because of their high affinity for a broad spectrum of structural targets, aptamers act very similar to antibodies.

Aptamers can be synthesized using standard phosphoramidite chemistry. In addition, RNA aptamers having more than approximately 30 nucleotides can be favorably synthesized in large amounts by in-vitro transcription. Selection, synthesis, and purification of aptamers are well-known to those skilled in the art.

Aptamers may carry functional labels in order to determine their binding quantity to the CXCL13 target. The labeling may be of any type described in the specification. Favorite labels have been characterized in detail in the course of the present description. The prior teaching of the present invention is considered as valid and without restrictions for all embodiments of labeling aptamers. The embodiments of prior teaching of the present invention are also considered as applicable to aptamers and their labeling if expedient.

In a preferred embodiment of the present invention, the nucleic acids are labeled with digoxigenin, biotin, fluorescence dyes well-known in the art, or radioactive isotopes.

Preferred isotopes for labeling nucleic acids in the scope of the invention are ³H, ¹⁴C, ³²P, ³³P, ³⁵S or ¹²⁵I, more preferred ³²P, ³³p, or ¹²⁵I.

Object of the present invention is also a method for ante mortem diagnosis of a TSE disease in a mammal comprising the incubation of a sample of a body fluid taken from the mammal with substances being specific to a surrogate marker of TSE, the detection of the specific incubation products, the determination of the surrogate marker concentration and based on it the diagnosis of TSE, wherein the body fluid which is taken from the mammal is selected form the group consisting of blood, serum, plasma, saliva, and urine, wherein substances being specific to CXCL13 are used for diagnosis of TSE, and wherein the stage of the TSE disease is differentiated by comparison with CXCL13 concentrations in samples of uninfected and/or infected mammals.

As already stated, TSE-related activities are shown by enhanced concentration levels of CXCL13 suggesting its use as surrogate marker. Surprisingly, it has been found that the CXCL13 concentration is clearly correlated to the progress of TSE disease. In contrast to Baker et al., 2004, J. NeuroVirology 10, 29-40, the remarkable CXCL13 mRNA increase in brain is not restricted to a period of 30 to 40 days after infection, and a final steep elevation at the terminal stage of CJD, but a direct proportion has be observed. Additionally surprising is the fact that the CXCL13 level in CNS is direct proportional to the CXCL13 level out of CNS, i.e. in the body fluids of blood, serum, plasma, saliva, and urine. In detail, CXCL13 seems to overcome the blood-brain-barrier at any stage of TSE disease. Its secretion only depends on the concentration gradient between brain and blood. Therefore, by determining the CXCL13 concentration and comparing it with a calibration curve, a possible TSE disease and the stage of the TSE disease can be easily diagnosed. Preferably, the calibration curve involves CXCL13 data from uninfected and infected mammals. It is to be understood that the calibration curve is determined in advance and scientifically and statistically proven regarding the different stages of a TSE disease. That means, each concentration measured can be exactly assigned to a reference magnitude of CXCL13 concentration on the calibration curve, the reference magnitude being in the range of a certain stage of TSE disease. Due to the current lack of TSE therapies, the inventive differential diagnosis may become vitally important in the evaluation of the infection risk of gregarious animals. In future, therapeutic approaches will be efficiently attuned to the etiopathology. Additionally, the ante mortem differential diagnosis can be favorably combined with other techniques and methods in order to confirm the TSE diagnosis.

All steps of ante mortem diagnosis of a TSE disease have been characterized in detail in the course of the present specification. The prior teaching of the present invention is considered as valid and without restrictions for all embodiments of ante mortem differential diagnosis of a TSE disease. Furthermore, the embodiments of prior teaching of the present invention are also applicable to the differential diagnosis if expedient.

In a preferred embodiment of the invention, in the case of a TSE disease the CXCL13 concentration in a sample of a mammal at the asymptomatic stage exceeds one and a half to four times, preferably twice to three times, the CXCL13 concentration in a sample of an uninfected mammal, and the CXCL13 concentration in a sample of a mammal at the clinical stage exceeds at least four times the CXCL13 concentration in a sample of an uninfected mammal.

Object of the present invention is also the use of substances being specific to CXCL13, preferably to bovine CXCL13, for ante mortem diagnosis of a TSE disease in a sample of blood, serum, plasma, saliva, or urine of a mammal showing no symptoms of TSE.

It is another object of the present invention to use the substances being specific to CXCL13, preferably to bovine CXCL13, for ante mortem differential diagnosis of a TSE disease in a sample of blood, serum, plasma, saliva, or urine of a mammal.

Another preferred object of the present invention relates to the use of the antibodies AF801, BAF801, MAB801 clone 53610, DCX130 part 892424, or DCX130 part 892425 as specific substances to CXCL13.

The invention also relates to a kit for use in ante mortem diagnosis of a TSE disease comprising substances being specific to CXCL13. In addition, the invention relates to a kit for use in ante mortem differential diagnosis of a TSE disease comprising substances being specific to CXCL13. In preferred embodiments of the invention, the kits comprise substances being specific to bovine CXCL13.

Example

The following example is provided by way of illustration and not by way of limitation. Within the example, standard reagents and buffers that are free from contaminating activities (whenever practical) are used.

C57B/6 mice were intraperitoneally infected with 20 µl diluted 10 % brain homogenates prepared from a scrapie strain 139A infected mouse. In addition, C57B1/6 mice were intracerebrally or orally infected with the identical amount and type of scrapie. As controls served the same number of mock-infected C57B1/6 mice similarly inoculated with 10 % brain homogenate from uninfected C57B1/6 mice. A small amount of blood (100 to 150 mL) was obtained from the tail vain of infected and mock-infected mice, respectively, on the following days post-infection (dpi): (1) 30, 59, 90, 100, 119, 153, and 189 dpi (terminal stage of disease), and (2) 25, 49, 75, 101, 126, 150, and 189 dpi. Immediately after cessation of breathing and cardiac arrest, 2 mL of blood were collected by puncture of the right cardiac ventricle. Serum was isolated and frozen by standard procedures and within three hours to prevent an artifactual increase in CXCL13 concentration. The determination of CXCL13 in the samples was performed by means of the Quantikine® Mouse BLC/BCA-1/CXCL13 immunoassay distributed by R&D Systems GmbH, Wiesbaden-Nordenstadt, Germany, catalog number MCX130, and in accordance with manufacturer's manual.

The results for the scrapie-infected mice concerning the CXCL13 concentrations are summarized in table 1.

The increase in CXCL13 concentration was independent on the mode of infection, and the CXCL13 concentration was clearly correlated to the stage of TSE disease: The higher the concentration the longer the mouse is infected. This proportion was also not affected by the margin of deviation which was relatively large in the scope of the present invention. The statistical imprecision is well-known to those skilled in the art since each recorded measurement reading is accompanied by a margin of deviation. In the present example, the following reasons have to be considered. First, each CXCL13 concentration resulted from the analysis of samples belonging to living organisms. The origin of sample, the cellular status, the environmental conditions of the taken body fluid etc. exerted an influence on the measurements. Secondly, the given concentration level of CXCL13 represented the averaged value calculated from the data of numerous samples of each TSE stage which is subjected to fluctuations itself, e.g. by the type of the infecting strain.

**Table 1: CXCL13 concentration in scrapie-infected mice at different stages of disease**

| **Stage of disease** | **Average CXCL13 concentration incl. standard deviation [pg/ml]** | **No. of subjects** |
|---|---|---|
| Uninfected | 264 ± 37 | 9 |
| Pre-clinical / asymptomatic | 707 ± 194 | 6 |
| Terminal sickened | 2078 ± 815 | 20 |

## Claims

1. A method for ante mortem diagnosis of a transmissible spongiform encephalopathy (TSE) disease in a mammal comprising the incubation of a sample of a body fluid taken from the mammal with substances being specific to a surrogate marker of TSE, the detection of the specific incubation products, the determination of the surrogate marker concentration and based on it the diagnosis of TSE, wherein the diagnosis of TSE is performed on a mammal showing no symptoms of TSE, wherein the body fluid which is taken from the mammal is selected from the group consisting of blood, serum, plasma, saliva, and urine, and wherein substances being specific to CXCL13 are used for diagnosis of TSE.

2. The method according to claim 1, wherein the TSE disease is scrapie, bovine spongiform encephalopathy (BSE), Creutzfeldt-Jakob disease (CJD), or variant Creutzfeldt-Jakob disease (vCJD).

3. The method according to claim 1, wherein the mammal is a human, cattle, sheep, goat, or deer.

4. The method according to claim 1, wherein substances being specific to bovine CXCL13 are used.

5. The method according to claim 1, wherein in the case of a TSE disease the CXCL13 concentration exceeds one and a half to four times, preferably twice to three times, the CXCL13 concentration in a sample of an uninfected mammal.

6. The method according to claim 1, wherein antibodies are used as specific substances to CXCL13 and the incubation products are detected by the labeling of the antibodies, preferably by ELISA, RIA, FIA, SPIA, or western blotting.

7. The method according to claim 6, wherein the antibodies are selected from the group consisting of goat polyclonal anti-human CXCL13 antibody AF801, biotinylated goat polyclonal anti-human CXCL13 antibody BAF801, mouse monoclonal anti-human CXCL13 antibody MAB801 clone 53610, mouse monoclonal anti-human CXCL13 antibody DCX130 part 892424, or mouse monoclonal anti-human CXCL13 antibody DCX130 part 892425.

8. The method according to claim 1, wherein nucleic acids are used as specific substances to CXCL13 and the incubation products are detected by the labeling of the nucleic acids.

9. A method for ante mortem diagnosis of a TSE disease in a mammal comprising the incubation of a sample of a body fluid taken from the mammal with substances being specific to a surrogate marker of TSE, the detection of the specific incubation products, the determination of the surrogate marker concentration and based on it the diagnosis of TSE, wherein the body fluid which is taken from the mammal is selected form the group consisting of blood, serum, plasma, saliva, and urine, wherein substances being specific to CXCL13 are used for diagnosis of TSE, and wherein the stage of the TSE disease is differentiated by comparison with CXCL13 concentrations in samples of uninfected and/or infected mammals.

10. The method according to claim 9, wherein in the case of a TSE disease the CXCL13 concentration in a sample of a mammal at the asymptomatic stage exceeds one and a half to four times, preferably twice to three times, the CXCL13 concentration in a sample of an uninfected mammal, and the CXCL13 concentration in a sample of a mammal at the clinical stage exceeds at least four times the CXCL13 concentration in a sample of an uninfected mammal.

11. Use of substances being specific to CXCL13, preferably to bovine CXCL13, for ante mortem diagnosis of a TSE disease in a sample of blood, serum, plasma, saliva, or urine of a mammal showing no symptoms of TSE.

12. Use of substances being specific to CXCL13, preferably to bovine CXCL13, for ante mortem differential diagnosis of a TSE disease in a sample of blood, serum, plasma, saliva, or urine of a mammal.

13. Use according to claim 11 or 12, wherein the specific substances are the antibodies AF801, BAF801, MAB801 clone 53610, DCX130 part 892424, or DCX130 part 892425.

14. Kit for use in ante mortem diagnosis of a TSE disease comprising substances being specific to CXCL13, preferably to bovine CXCL13.
